# EUROPEAN PATENT APPLICATION

(11) **EP 4 124 324 A1**
(43) Date of publication of application: **01.02.2023**
(21) Application number: 22187052.0
(22) Date of filing: 05.06.2018
(51) Int. Cl.: A61F 13/00, A61F 13/02, A61M 1/00

(54) **PEEL AND PLACE DRESSING FOR THICK EXUDATE AND INSTILLATION**

(30) Priority: 07.06.2017 US 201762516550 P; 07.06.2017 US 201762516566 P; 07.06.2017 US 201762516540 P; 29.09.2017 US 201762565754 P; 24.10.2017 US 201762576498 P; 30.11.2017 US 201762592950 P; 04.01.2018 US 201862613494 P
(62) Divisional of application: 18733492.5
(71) Applicant: 3M Innovative Properties Company, St. Paul, MN 55133-3427 (US)
(72) Inventor: ROBINSON, Timothy Mark, Shillingtone (GB); LOCKE, Christopher Brian, Bournemouth (GB)
(74) Representative: Simmons & Simmons

(57) **Abstract**

A multi-layer dressing for treating tissue with negative pressure, instillation, or both. In some embodiments a first layer may be formed from reticulated foam having a series of holes. A second layer disposed adjacent to the first layer may be formed from a perforated polymer. The dressing may optionally include a third layer formed from a soft polymer, such as a silicone gel. The third layer may also have perforations or apertures. The third layer is generally oriented to face a tissue site, and may be disposed adjacent to the first layer so that the first layer is disposed between the third layer and the first layer. The perforations or apertures in the third layer may be registered with one or more perforations in the first layer.

## Description

### RELATED APPLICATION

This application claims the benefit, under 35 U.S.C. § 119(e), of the filing of U.S. Provisional Patent Application serial number 62/613,494, entitled "PEEL AND PLACE DRESSING FOR THICK EXUDATE AND INSTILLATION," filed January 4, 2018; U.S. Provisional Patent Application serial number 62/592,950, entitled "MULTI-LAYER WOUND FILLER FOR EXTENDED WEAR TIME," filed November 30, 2017; U.S. Provisional Patent Application serial number 62/576,498, entitled "SYSTEMS, APPARATUSES, AND METHODS FOR NEGATIVE-PRESSURE TREATMENT WITH REDUCED TISSUE IN-GROWTH," filed October 24, 2017; U.S. Provisional Patent Application serial number 62/565,754, entitled "COMPOSITE DRESSINGS FOR IMPROVED GRANULATION AND REDUCED MACERATION WITH NEGATIVE-PRESSURE TREATMENT," filed September 29, 2017; U.S. Provisional Patent Application serial number 62/516,540, entitled "TISSUE CONTACT INTERFACE," filed June 7, 2017; U.S. Provisional Patent Application serial number 62/516,550, entitled "COMPOSITE DRESSINGS FOR IMPROVED GRANULATION AND REDUCED MACERATION WITH NEGATIVE-PRESSURE TREATMENT" filed June 7, 2017; and U.S. Provisional Patent Application serial number 62/516,566, entitled "COMPOSITE DRESSINGS FOR IMPROVED GRANULATION AND REDUCED MACERATION WITH NEGATIVE-PRESSURE TREATMENT" filed June 7, 2017, each of which is incorporated herein by reference for all purposes.

### TECHNICAL FIELD

The invention set forth in the appended claims relates generally to tissue treatment systems and more particularly, but without limitation, to systems, apparatuses, and methods for treating tissue in a negative-pressure therapy environment.

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times.

There is also widespread acceptance that cleansing a tissue site can be highly beneficial for new tissue growth. For example, a wound can be washed out with a stream of liquid solution, or a cavity can be washed out using a liquid solution for therapeutic purposes. These practices are commonly referred to as "irrigation" and "lavage" respectively. "Instillation" is another practice that generally refers to a process of slowly introducing fluid to a tissue site and leaving the fluid for a prescribed period of time before removing the fluid. For example, instillation of topical treatment solutions over a wound bed can be combined with negative-pressure therapy to further promote wound healing by loosening soluble contaminants in a wound bed and removing infectious material. As a result, soluble bacterial burden can be decreased, contaminants removed, and the wound cleansed.

While the clinical benefits of negative-pressure therapy and/or instillation therapy are widely known, improvements to therapy systems, components, and processes may benefit healthcare providers and patients.

### BRIEF SUMMARY

New and useful systems, apparatuses, and methods for treating tissue in a negative-pressure therapy environment are set forth in the appended claims. Illustrative embodiments are also provided to enable a person skilled in the art to make and use the claimed subject matter.

For example, in some embodiments, a dressing for treating tissue may comprise a first layer of porous manifolding material. The first layer may be formed from reticulated foam in some examples. A series of holes may be formed in the first layer. The holes may have a diameter of about 2 millimeters to about 10 millimeters in some examples, and may have a center spacing of about 2 millimeters to about 10 millimeters in some examples. A second layer may be disposed adjacent to the first layer. The second layer may be formed from a perforated polymer. A hydrophobic polymer may be advantageous for some embodiments. A thickness of 25 to 50 microns may be suitable for some embodiments of the second layer. In some embodiments, the perforations may be slots. The width of the slots may be between 0.5 millimeters and 2 millimeters in some examples, and may have a length of about 3 millimeters to about 5 millimeters in some examples. The perforations may or may not be registered with holes in the first layer. The perforations may be long or crossed fenestrations registered or aligned with holes, and the perforations can act as flaps to prevent granulation tissue from contacting the sides of the holes. The second layer is generally oriented to face a tissue site. The dressing may optionally include a third layer formed from a soft polymer. The soft polymer may be, for example, a silicone gel. A coating weight of about 450 grams per square meter may be suitable for the silicone gel in some configurations. Other example polymers that may be suitable include polyurethane, hydrocolloids, and acrylics. The third layer may also have perforations or apertures. The third layer is generally oriented to face a tissue site, and may be disposed adjacent to the first layer so that the first layer is disposed between the third layer and the first layer. The perforations or apertures in the third layer may be registered with one or more perforations in the first layer. Registering or aligning the perforations can reduce pressure drop effects of thick exudate and permit instillation solution to rapidly move through the dressing to the tissue. Perforations and apertures in the first layer and the third layer may be sized differently depending on location in some embodiments. For example, smaller perforations toward the edge of a dressing may direct instillation solution toward a central portion of the dressing and away from a perimeter of the dressing.

More generally, an apparatus for treating a tissue site with negative pressure may be a dressing comprising a first layer and a second layer disposed adjacent to each other in a stacked relationship, and a cover disposed over the first layer. The first layer may comprise a manifold having a plurality of through-holes, and the second layer may comprise a polymer film having a plurality of fluid restrictions that are configured to expand in response to a pressure gradient across the polymer film. The cover may comprise or consist essentially of a polymer drape in some examples. In some embodiments, each of the plurality of through-holes may have an effective diameter between about 2 millimeters and about 10 millimeters, and may be spaced between about 2 millimeters and about 10 millimeters on center. In some embodiments, the through-holes may be arranged as an array. The through-holes may be offset from the fluid restrictions, or may be registered with at least some of the fluid restrictions.

Additionally, the dressing may comprise a third layer in some embodiments. The third layer may be disposed adjacent to the second layer, and may comprise or consist essentially of a gel layer having a plurality of apertures. The gel layer may be hydrophobic in some embodiments. A polymer gel having a coat weight of about 250 grams per square centimeter may be suitable for some embodiments of the third layer.

Objectives, advantages, and a preferred mode of making and using the claimed subject matter may be understood best by reference to the accompanying drawings in conjunction with the following detailed description of illustrative embodiments.

### BRIEF DESCRIPTION OF THE DRAWINGS

Figure 1 is a functional block diagram of an example embodiment of a therapy system that can provide negative-pressure treatment and instillation treatment in accordance with this specification;
Figure 2 is a graph illustrating additional details of example pressure control modes that may be associated with some embodiments of the therapy system of Figure 1;
Figure 3 is a graph illustrating additional details that may be associated with another example pressure control mode in some embodiments of the therapy system of Figure 1;
Figure 4 is an assembly view of an example of a dressing that may be associated with some embodiments of the therapy system of Figure 1;
Figure 5 is a schematic view of an example of a layer that may be associated with some embodiments of the dressing of Figure 4;
Figure 6 is an assembly view of another example of a dressing that may be associated with some embodiments of the therapy system of Figure 1;
Figure 7 is a schematic view of an example configuration of apertures that may be associated with some embodiments of a layer in the dressing of Figure 6;
Figure 8 is a schematic view of the example layer of Figure 7 overlaid on the example layer of Figure 5;
Figure 9 is a top view of another example a layer that may be associated with some embodiments of the dressing of Figure 1;
Figure 10 and Figure 11 illustrate other example configurations of valves that may be associated with various layers in the dressing of Figure 1;
Figure 12 is a schematic section view of an example of the dressing of Figure
Figure 13 is a schematic section view of another example of the dressing of Figure 1;
Figure 14 is a plan view of an example of a manifold layer having perforations that may be associated with some embodiments of the dressing of Figure 1;
Figure 15 and Figure 16 are plan views of other examples of perforations in a manifold layer that may be associated with some embodiments of the dressing of Figure 1;
Figure 17 is a plan view of another example of perforations in a manifold layer that may be associated with some embodiments of the dressing of Figure 1; and
Figure 18 is a chart illustrating details that may be associated with an example method of operating the therapy system of Figure 1.

### DESCRIPTION OF EXAMPLE EMBODIMENTS

The following description of example embodiments provides information that enables a person skilled in the art to make and use the subject matter set forth in the appended claims, but may omit certain details already well-known in the art. The following detailed description is, therefore, to be taken as illustrative and not limiting.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

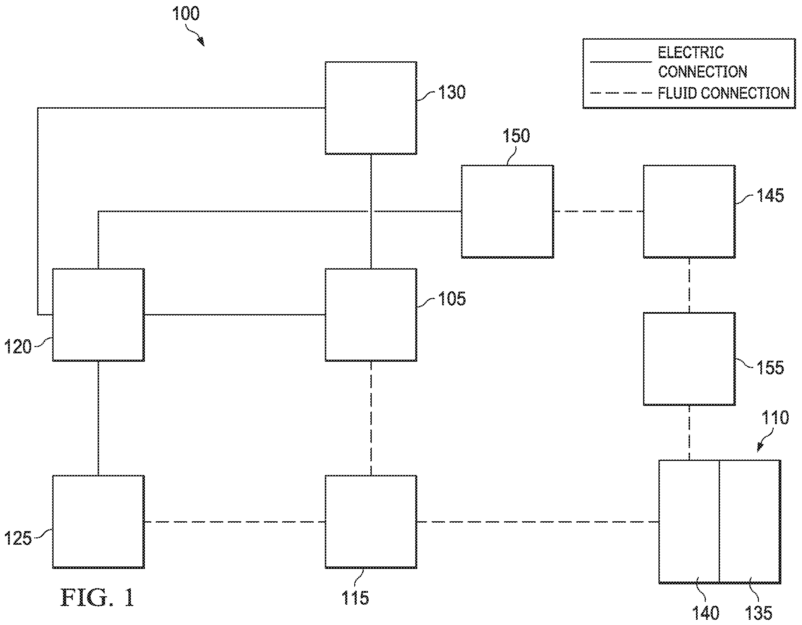

Figure 1 is a simplified functional block diagram of an example embodiment of a therapy system 100 that can provide negative-pressure therapy with instillation of topical treatment solutions to a tissue site in accordance with this specification.

The term "tissue site" in this context broadly refers to a wound, defect, or other treatment target located on or within tissue, including but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted.

The therapy system 100 may include a source or supply of negative pressure, such as a negative-pressure source 105, a dressing 110, a fluid container, such as a container 115, and a regulator or controller, such as a controller 120, for example. Additionally, the therapy system 100 may include sensors to measure operating parameters and provide feedback signals to the controller 120 indicative of the operating parameters. As illustrated in Figure 1, for example, the therapy system 100 may include a first sensor 125 and a second sensor 130 coupled to the controller 120. As illustrated in the example of Figure 1, the dressing 110 may comprise or consist essentially of a tissue interface 135, a cover 140, or both in some embodiments.

The therapy system 100 may also include a source of instillation solution. For example, a solution source 145 may be fluidly coupled to the dressing 110, as illustrated in the example embodiment of Figure 1. The solution source 145 may be fluidly coupled to a positive-pressure source such as the positive-pressure source 150, a negative-pressure source such as the negative-pressure source 105, or both in some embodiments. A regulator, such as an instillation regulator 155, may also be fluidly coupled to the solution source 145 and the dressing 110 to ensure proper dosage of instillation solution (e.g. saline) to a tissue site. For example, the instillation regulator 155 may comprise a piston that can be pneumatically actuated by the negative-pressure source 105 to draw instillation solution from the solution source during a negative-pressure interval and to instill the solution to a dressing during a venting interval. Additionally or alternatively, the controller 120 may be coupled to the negative-pressure source 105, the positive-pressure source 150, or both, to control dosage of instillation solution to a tissue site. In some embodiments, the instillation regulator 155 may also be fluidly coupled to the negative-pressure source 105 through the dressing 110, as illustrated in the example of Figure 1.

Some components of the therapy system 100 may be housed within or used in conjunction with other components, such as sensors, processing units, alarm indicators, memory, databases, software, display devices, or user interfaces that further facilitate therapy. For example, in some embodiments, the negative-pressure source 105 may be combined with the solution source 145, the controller 120 and other components into a therapy unit.

In general, components of the therapy system 100 may be coupled directly or indirectly. For example, the negative-pressure source 105 may be directly coupled to the container 115, and may be indirectly coupled to the dressing 110 through the container 115. Coupling may include fluid, mechanical, thermal, electrical, or chemical coupling (such as a chemical bond), or some combination of coupling in some contexts. For example, the negative-pressure source 105 may be electrically coupled to the controller 120, and may be fluidly coupled to one or more distribution components to provide a fluid path to a tissue site. In some embodiments, components may also be coupled by virtue of physical proximity, being integral to a single structure, or being formed from the same piece of material.

A distribution component is preferably detachable, and may be disposable, reusable, or recyclable. The dressing 110 and the container 115 are illustrative of distribution components. A fluid conductor is another illustrative example of a distribution component. A "fluid conductor," in this context, broadly includes a tube, pipe, hose, conduit, or other structure with one or more lumina or open pathways adapted to convey a fluid between two ends. Typically, a tube is an elongated, cylindrical structure with some flexibility, but the geometry and rigidity may vary. Moreover, some fluid conductors may be molded into or otherwise integrally combined with other components. Distribution components may also include or comprise interfaces or fluid ports to facilitate coupling and de-coupling other components. In some embodiments, for example, a dressing interface may facilitate coupling a fluid conductor to the dressing 110. For example, such a dressing interface may be a SENSAT.R.A.C.^{™} Pad available from Kinetic Concepts, Inc. of San Antonio, Texas.

A negative-pressure supply, such as the negative-pressure source 105, may be a reservoir of air at a negative pressure, or may be a manual or electrically-powered device, such as a vacuum pump, a suction pump, a wall suction port available at many healthcare facilities, or a micro-pump, for example. "Negative pressure" generally refers to a pressure less than a local ambient pressure, such as the ambient pressure in a local environment external to a sealed therapeutic environment. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. References to increases in negative pressure typically refer to a decrease in absolute pressure, while decreases in negative pressure typically refer to an increase in absolute pressure. While the amount and nature of negative pressure applied to a tissue site may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between -50 mm Hg (-6.7 kPa) and -300 mm Hg (-39.9 kPa).

The container 115 is representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with negative-pressure therapy.

A controller, such as the controller 120, may be a microprocessor or computer programmed to operate one or more components of the therapy system 100, such as the negative-pressure source 105. In some embodiments, for example, the controller 120 may be a microcontroller, which generally comprises an integrated circuit containing a processor core and a memory programmed to directly or indirectly control one or more operating parameters of the therapy system 100. Operating parameters may include the power applied to the negative-pressure source 105, the pressure generated by the negative-pressure source 105, or the pressure distributed to the tissue interface 135, for example. The controller 120 is also preferably configured to receive one or more input signals, such as a feedback signal, and programmed to modify one or more operating parameters based on the input signals.

Sensors, such as the first sensor 125 and the second sensor 130, are generally known in the art as any apparatus operable to detect or measure a physical phenomenon or property, and generally provide a signal indicative of the phenomenon or property that is detected or measured. For example, the first sensor 125 and the second sensor 130 may be configured to measure one or more operating parameters of the therapy system 100. In some embodiments, the first sensor 12.5 may be a transducer configured to measure pressure in a pneumatic pathway and convert the measurement to a signal indicative of the pressure measured. In some embodiments, for example, the first sensor 125 may be a piezo-resistive strain gauge. The second sensor 130 may optionally measure operating parameters of the negative-pressure source 105, such as a voltage or current, in some embodiments. Preferably, the signals from the first sensor 125 and the second sensor 130 are suitable as an input signal to the controller 120, but some signal conditioning may be appropriate in some embodiments. For example, the signal may need to be filtered or amplified before it can be processed by the controller 120. Typically, the signal is an electrical signal, but may be represented in other forms, such as an optical signal.

The tissue interface 135 can be generally adapted to partially or fully contact a tissue site. The tissue interface 135 may take many forms, and may have many sizes, shapes, or thicknesses depending on a variety of factors, such as the type of treatment being implemented or the nature and size of a tissue site. For example, the size and shape of the tissue interface 135 may be adapted to the contours of deep and irregular shaped tissue sites. Moreover, any or all of the surfaces of the tissue interface 135 may have projections or an uneven, course, or jagged profile that can induce strains and stresses on a tissue site, which can promote granulation at the tissue site.

In some embodiments, the cover 140 may provide a bacterial barrier and protection from physical trauma. The cover 140 may also be constructed from a material that can reduce evaporative losses and provide a fluid seal between two components or two environments, such as between a therapeutic environment and a local external environment. The cover 140 may comprise or consist of, for example, an elastomeric film or membrane that can provide a seal adequate to maintain a negative pressure at a tissue site for a given negative-pressure source. The cover 140 may have a high moisture-vapor transmission rate (MVTR) in some applications. For example, the MVTR may be at least 250 grams per square meter per twenty-four hours in some embodiments, measured using an upright cup technique according to ASTM E96/E96M Upright Cup Method at 38°C and 10% relative humidity (RH). In some embodiments, an MVTR up to 5,000 grams per square meter per twenty-four hours may provide may provide effective breathability and mechanical properties.

In some example embodiments, the cover 140 may be a polymer drape, such as a polyurethane film, that is permeable to water vapor but impermeable to liquid. Such drapes typically have a thickness in the range of 25-50 microns. For permeable materials, the permeability generally should be low enough that a desired negative pressure may be maintained. The cover 140 may comprise, for example, one or more of the following materials: polyurethane (PU), such as hydrophilic polyurethane; cellulosics; hydrophilic polyamides; polyvinyl alcohol; polyvinyl pyrrolidone; hydrophilic acrylics; silicones, such as hydrophilic silicone elastomers; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; ethylene vinyl acetate (EVA); co-polyester; and polyether block polymide copolymers. Such materials are commercially available as, for example, Tegaderm^{®} drape, commercially available from 3M Company, Minneapolis Minnesota; polyurethane (PU) drape, commercially available from Avery Dennison Corporation, Pasadena, California; polyether block polyamide copolymer (PEBAX), for example, from Arkema S.A., Colombes, France; and Inspire 2301 and Inpsire 2327 polyurethane films, commercially available from Expopack Advanced Coatings, Wrexham, United Kingdom. In some embodiments, the cover 140 may comprise INSPIRE 2301 having an MVTR (upright cup technique) of 2600 g/m²/24 hours and a thickness of about 30 microns.

An attachment device may be used to attach the cover 140 to an attachment surface, such as undamaged epidermis, a gasket, or another cover. The attachment device may take many forms. For example, an attachment device may be a medically-acceptable, pressure-sensitive adhesive configured to bond the cover 140 to epidermis around a tissue site. In some embodiments, for example, some or all of the cover 140 may be coated with an adhesive, such as an acrylic adhesive, which may have a coating weight between 25-65 grams per square meter (g.s.m.). In some embodiments, adhesive may be disposed in a margin around the cover 140. Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

The solution source 145 may also be representative of a container, canister, pouch, bag, or other storage component, which can provide a solution for instillation therapy. Compositions of solutions may vary according to a prescribed therapy, but examples of solutions that may be suitable for some prescriptions include hypochlorite-based solutions, silver nitrate (0.5%), sulfur-based solutions, biguanides, cationic solutions, and isotonic solutions.

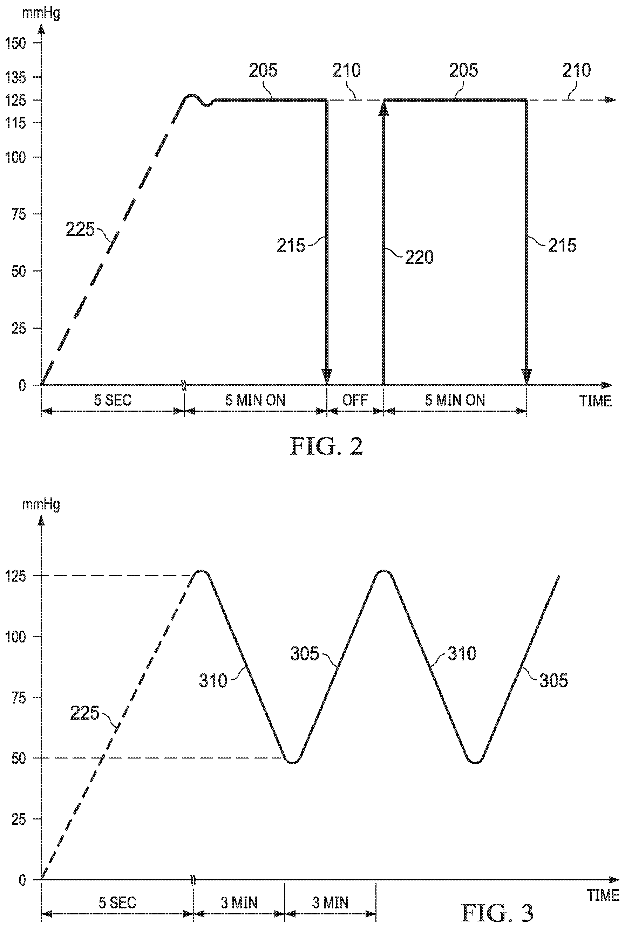

Figure 2 is a graph illustrating additional details of an example control mode that may be associated with some embodiments of the controller 120. In some embodiments, the controller 120 may have a continuous pressure mode, in which the negative-pressure source 105 is operated to provide a constant target negative pressure, as indicated by line 205 and line 210, for the duration of treatment or until manually deactivated. Additionally or alternatively, the controller may have an intermittent pressure mode, as illustrated in the example of Figure 2. In Figure 2, the x-axis represents time, and the y-axis represents negative pressure generated by the negative-pressure source 105 over time. In the example of Figure 2, the controller 120 can operate the negative-pressure source 105 to cycle between a target pressure and atmospheric pressure. For example, the target pressure may be set at a value of 125 mmHg, as indicated by line 205, for a specified period of time (e.g., 5 min), followed by a specified period of time (e.g., 2 min) of deactivation, as indicated by the gap between the solid lines 215 and 220. The cycle can be repeated by activating the negative-pressure source 105, as indicated by line 220, which can form a square wave pattern between the target pressure and atmospheric pressure.

In some example embodiments, the increase in negative-pressure from ambient pressure to the target pressure may not be instantaneous. For example, the negative-pressure source 105 and the dressing 110 may have an initial rise time, as indicated by the dashed line 225. The initial rise time may vary depending on the type of dressing and therapy equipment being used. For example, the initial rise time for one therapy system may be in a range of about 20-30 mmHg/second and in a range of about 5-10 mmHg/second for another therapy system. If the therapy system 100 is operating in an intermittent mode, the repeating rise time as indicated by the solid line 220 may be a value substantially equal to the initial rise time as indicated by the dashed line 225.

Figure 3 is a graph illustrating additional details that may be associated with another example pressure control mode in some embodiments of the therapy system 100. In Figure 3, the x-axis represents time and the y-axis represents negative pressure generated by the negative-pressure source 105. The target pressure in the example of Figure 3 can vary with time in a dynamic pressure mode. For example, the target pressure may vary in the form of a triangular waveform, varying between a minimum and maximum negative pressure of 50-125 mmHg with a rise time 305 set at a rate of +25 mmHg/min. and a descent time 310 set at -25 mmHg/min, respectively. In other embodiments of the therapy system 100, the triangular waveform may vary between negative pressure of 25-125 mmHg with a rise time 305 set at a rate of +30 mmHg/min and a descent time 310 set at -30 mmHg/min.

In some embodiments, the controller 120 may control or determine a variable target pressure in a dynamic pressure mode, and the variable target pressure may vary between a maximum and minimum pressure value that may be set as an input prescribed by an operator as the range of desired negative pressure. The variable target pressure may also be processed and controlled by the controller 120, which can vary the target pressure according to a predetermined waveform, such as a triangular waveform, a sine waveform, or a saw-tooth waveform. In some embodiments, the waveform may be set by an operator as the predetermined or time-varying negative pressure desired for therapy.

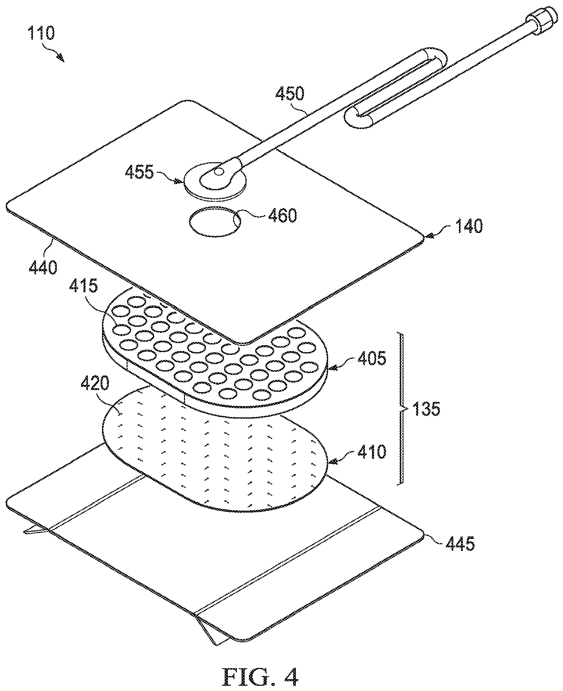

Figure 4 is an assembly view of an example of the dressing 110 of Figure 1, illustrating additional details that may be associated with some embodiments in which the tissue interface 135 comprises more than one layer. In the example of Figure 4, the tissue interface 135 comprises a first layer 405 and a second layer 410. In some embodiments, the first layer 405 may be disposed adjacent to the second layer 410. For example, the first layer 405 and the second layer 410 may be stacked so that the first layer 405 is in contact with the second layer 410. The first layer 405 may also be bonded to the second layer 410 in some embodiments.

The first layer 405 generally comprises or consists essentially of a manifold or a manifold layer having a plurality of perforations 415. The first layer 405 can provide a means for collecting or distributing fluid across the tissue interface 135 under pressure. For example, the first layer 405 may be adapted to receive negative pressure from a source and distribute negative pressure through multiple apertures across the tissue interface 135, which may have the effect of collecting fluid from across a tissue site and drawing the fluid toward the source. In some embodiments, the fluid path may be reversed or a secondary fluid path may be provided to facilitate delivering fluid, such as from a source of instillation solution, across the tissue interface 135.

In some illustrative embodiments, the pathways of the first layer 405 may be interconnected to improve distribution or collection of fluids. In some illustrative embodiments, the first layer 405 may comprise or consist essentially of a porous material having interconnected fluid pathways. For example, open-cell foam, porous tissue collections, and other porous material such as gauze or felted mat generally include pores, edges, and/or walls adapted to form interconnected fluid channels. Other suitable materials may include a 3D textile (Baltex, Muller, Heathcoates), non-woven (Libeltex, Freudenberg), a 3D polymeric structure (molded polymers, embossed and formed films, and fusion bonded films [Supracore]), and mesh, for example. Liquids, gels, and other foams may also include or be cured to include apertures and fluid pathways. In some embodiments, the first layer 405 may additionally or alternatively comprise projections that form interconnected fluid pathways. For example, the first layer 405 may be molded to provide surface projections that define interconnected fluid pathways. Any or all of the surfaces of the first layer 405 may have an uneven, coarse, or jagged profile

In some embodiments, the first layer 405 may comprise or consist essentially of reticulated foam having pore sizes and free volume that may vary according to needs of a prescribed therapy. For example, reticulated foam having a free volume of at least 90% may be suitable for many therapy applications, and foam having an average pore size in a range of 400-600 microns may be particularly suitable for some types of therapy. The tensile strength of the first layer 405 may also vary according to needs of a prescribed therapy. For example, the tensile strength of the first layer 405 may be increased for instillation of topical treatment solutions. The 25% compression load deflection of the first layer 405 may be at least 0.35 pounds per square inch, and the 65% compression load deflection may be at least 0.43 pounds per square inch. In some embodiments, the tensile strength of the first layer 405 may be at least 10 pounds per square inch. The first layer 405 may have a tear strength of at least 2.5 pounds per inch. In some embodiments, the first layer 405 may be foam comprised of polyols such as polyester or polyether, isocyanate such as toluene diisocyanate, and polymerization modifiers such as amines and tin compounds. In one non-limiting example, the first layer 405 may be a reticulated polyurethane foam such as used in GRANUFOAM^{™} dressing or V.A.C. VERAFLO^{™} dressing, both available from Kinetic Concepts, Inc. of San Antonio, Texas.

The first layer 405 generally has a first planar surface and a second planar surface opposite the first planar surface. The thickness of the first layer 405 between the first planar surface and the second planar surface may also vary according to needs of a prescribed therapy. For example, the thickness of the first layer 405 may be decreased to relieve stress on other layers and to reduce tension on peripheral tissue. The thickness of the first layer 405 can also affect the conformability of the first layer 405. In some embodiments, a thickness in a range of about 5 millimeters to 10 millimeters may be suitable.

The second layer 410 may be a diverter layer, comprising or consisting essentially of a means for controlling or managing fluid flow. In some embodiments, the second layer 410 may comprise or consist essentially of a liquid-impermeable, elastomeric material. For example, the second layer 410 may comprise or consist essentially of a polymer film. The second layer 410 may also have a smooth or matte surface texture in some embodiments. A glossy or shiny finish better or equal to a grade B3 according to the SPI (Society of the Plastics Industry) standards may be particularly advantageous for some applications. In some embodiments, variations in surface height may be limited to acceptable tolerances. For example, the surface of the second layer may have a substantially flat surface, with height variations limited to 0.2 millimeters over a centimeter.

In some embodiments, the second layer 410 may be hydrophobic. The hydrophobicity of the second layer 410 may vary, but may have a contact angle with water of at least ninety degrees in some embodiments. In some embodiments the second layer 410 may have a contact angle with water of no more than 150 degrees. For example, in some embodiments, the contact angle of the second layer 410 may be in a range of at least 90 degrees to about 120 degrees, or in a range of at least 120 degrees to 150 degrees. Water contact angles can be measured using any standard apparatus. Although manual goniometers can be used to visually approximate contact angles, contact angle measuring instruments can often include an integrated system involving a level stage, liquid dropper such as a syringe, camera, and software designed to calculate contact angles more accurately and precisely, among other things. Non-limiting examples of such integrated systems may include the FTÅ125, FTÅ200, FTÅ2000, and FTÅ4000 systems, all commercially available from First Ten Angstroms, Inc., of Portsmouth, VA, and the DTA25, DTA30, and DTA100 systems, all commercially available from Kruss GmbH of Hamburg, Germany. Unless otherwise specified, water contact angles herein are measured using deionized and distilled water on a level sample surface for a sessile drop added from a height of no more than 5 cm in air at 20-25°C and 20-50% relative humidity. Contact angles reported herein represent averages of 5-9 measured values, discarding both the highest and lowest measured values. The hydrophobicity of the second layer 410 may be further enhanced with a hydrophobic coating of other materials, such as silicones and fluorocarbons, either as coated from a liquid, or plasma coated.

The second layer 410 may also be suitable for welding to other layers, including the first layer 405. For example, the second layer 410 may be adapted for welding to polyurethane foams using heat, radio frequency (RF) welding, or other methods to generate heat such as ultrasonic welding. RF welding may be particularly suitable for more polar materials, such as polyurethane, polyamides, polyesters and acrylates. Sacrificial polar interfaces may be used to facilitate RF welding of less polar film materials, such as polyethylene.

The area density of the second layer 410 may vary according to a prescribed therapy or application. In some embodiments, an area density of less than 40 grams per square meter may be suitable, and an area density of about 20-30 grams per square meter may be particularly advantageous for some applications.

In some embodiments, for example, the second layer 410 may comprise or consist essentially of a hydrophobic polymer, such as a polyethylene film. The simple and inert structure of polyethylene can provide a surface that interacts little, if any, with biological tissues and fluids, providing a surface that may encourage the free flow of liquids and low adherence, which can be particularly advantageous for many applications. Other suitable polymeric films include polyurethanes, acrylics, polyolefin (such as cyclic olefin copolymers), polyacetates, polyamides, polyesters, copolyesters, PEBAX block copolymers, thermoplastic elastomers, thermoplastic vulcanizates, polyethers, polyvinyl alcohols, polypropylene, polymethylpentene, polycarbonate, styreneics, silicones, fluoropolymers, and acetates. A thickness between 20 microns and 100 microns may be suitable for many applications. Films may be clear, colored, or printed. More polar films suitable for laminating to a polyethylene film include polyamide, co-polyesters, ionomers, and acrylics. To aid in the bond between a polyethylene and polar film, tie layers may be used, such as ethylene vinyl acetate, or modified polyurethanes. An ethyl methyl acrylate (EMA) film may also have suitable hydrophobic and welding properties for some configurations.

As illustrated in the example of Figure 4, the second layer 410 may have one or more fluid restrictions 420, which can be distributed uniformly or randomly across the second layer 410. The fluid restrictions 420 may be bi-directional and pressure-responsive. For example, each of the fluid restrictions 420 generally may comprise or consist essentially of an elastic passage that is normally unstrained to substantially reduce liquid flow, and can expand or open in response to a pressure gradient. In some embodiments, the fluid restrictions 420 may comprise or consist essentially of perforations in the second layer 410. Perforations may be formed by removing material from the second layer 410. For example, perforations may be formed by cutting through the second layer 410, which may also deform the edges of the perforations in some embodiments. In the absence of a pressure gradient across the perforations, the passages may be sufficiently small to form a seal or fluid restriction, which can substantially reduce or prevent liquid flow. Additionally or alternatively, one or more of the fluid restrictions 420 may be an elastomeric valve that is normally closed when unstrained to substantially prevent liquid flow, and can open in response to a pressure gradient. A fenestration in the second layer 410 may be a suitable valve for some applications. Fenestrations may also be formed by removing material from the second layer 410, but the amount of material removed and the resulting dimensions of the fenestrations may be up to an order of magnitude less than perforations, and may not deform the edges.

For example, some embodiments of the fluid restrictions 420 may comprise or consist essentially of one or more slits, slots or combinations of slits and slots in the second layer 410. In some examples, the fluid restrictions 420 may comprise or consist of linear slots having a length less than 6 millimeters and a width less than 3 millimeters. The length may be at least 2 millimeters, and the width may be at least 0.4 millimeters in some embodiments. A length of about 3 millimeters to about 5 millimeters and a width of about 0.5 millimeters to about 2 millimeters may be particularly suitable for many applications, and a tolerance of about 0.1 millimeter may also be acceptable. Such dimensions and tolerances may be achieved with a laser cutter, for example. Slots of such configurations may function as imperfect valves that substantially reduce liquid flow in a normally closed or resting state. For example, such slots may form a flow restriction without being completely closed or sealed. The slots can expand or open wider in response to a pressure gradient to allow increased liquid flow.

In the example of Figure 4, the dressing 110 may further include an attachment device, such as an adhesive 440. The adhesive 440 may be, for example, a medically-acceptable, pressure-sensitive adhesive that extends about a periphery, a portion, or the entire cover 140. In some embodiments, for example, the adhesive 440 may be an acrylic adhesive having a coating weight between 25-65 grams per square meter (g.s.m.). In the example of Figure 4, the cover comprises a margin that extends beyond the first layer 405 and the second layer 410, and the adhesive 440 may be disposed in the margin. Thicker adhesives, or combinations of adhesives, may be applied in some embodiments to improve the seal and reduce leaks. In some embodiments, such a layer of the adhesive 440 may be continuous or discontinuous. Discontinuities in the adhesive 440 may be provided by apertures or holes (not shown) in the adhesive 440. The apertures or holes in the adhesive 440 may be formed after application of the adhesive 440 or by coating the adhesive 440 in patterns on a carrier layer, such as, for example, a side of the cover 140. Apertures or holes in the adhesive 440 may also be sized to enhance the moisture-vapor transfer rate of the dressing 110 in some example embodiments.

As illustrated in the example of Figure 4, in some embodiments, the dressing 110 may include a release liner 445 to protect the adhesive 440 prior to use. The release liner 445 may also provide stiffness, which can facilitate deployment of the dressing 110. The release liner 445 may be, for example, a casting paper, a film, or polyethylene. Further, in some embodiments, the release liner 445 may be a polyester material such as polyethylene terephthalate (PET), or similar polar semi-crystalline polymer. The use of a polar semi-crystalline polymer for the release liner 445 may substantially preclude wrinkling or other deformation of the dressing 110. For example, the polar semi-crystalline polymer may be highly orientated and resistant to softening, swelling, or other deformation that may occur when brought into contact with components of the dressing 110, or when subjected to temperature or environmental variations, or sterilization. Further, a release agent may be disposed on a side of the release liner 445 that is configured to contact the second layer 410. For example, the release agent may be a silicone coating and may have a release factor suitable to facilitate removal of the release liner 445 by hand and without damaging or deforming the dressing 110. In some embodiments, the release agent may be a fluorocarbon or a fluorosilicone, for example. In other embodiments, the release liner 445 may be uncoated or otherwise used without a release agent.

Figure 4 also illustrates one example of a fluid conductor 450 and a dressing interface 455. As shown in the example of Figure 4, the fluid conductor 450 may be a flexible tube, which can be fluidly coupled on one end to the dressing interface 455. The dressing interface 455 may be an elbow connector, as shown in the example of Figure 4, which can be placed over an aperture 460 in the cover 140 to provide a fluid path between the fluid conductor 450 and the tissue interface 135.

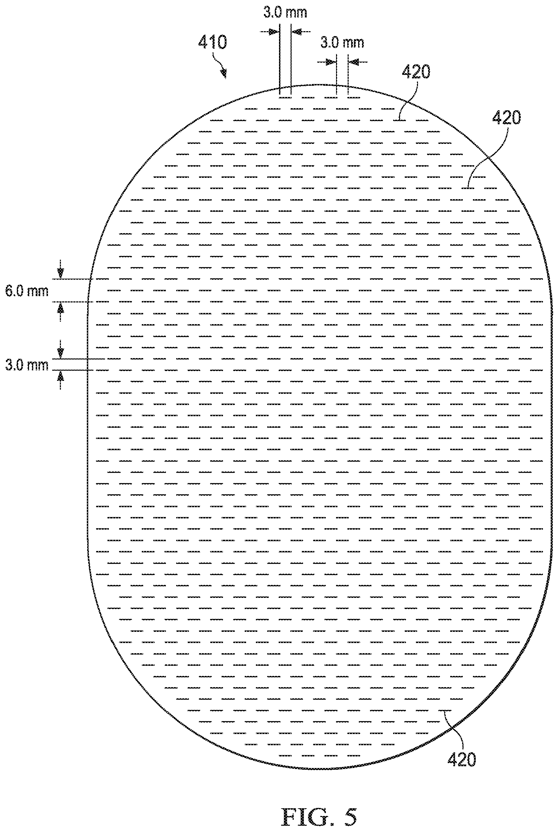

Figure 5 is a schematic view of an example of the second layer 410, illustrating additional details that may be associated with some embodiments. As illustrated in the example of Figure 5, the fluid restrictions 420 may each consist essentially of one or more linear slots having a length of about 3 millimeters. Figure 5 additionally illustrates an example of a uniform distribution pattern of the fluid restrictions 420. In Figure 5, the fluid restrictions 420 are substantially coextensive with the second layer 410, and are distributed across the second layer 410 in a grid of parallel rows and columns, in which the slots are also mutually parallel to each other. In some embodiments, the rows may be spaced about 3 millimeters on center, and the fluid restrictions 420 within each of the rows may be spaced about 3 millimeters on center as illustrated in the example of Figure 5. The fluid restrictions 420 in adjacent rows may be aligned or offset. For example, adjacent rows may be offset, as illustrated in Figure 5, so that the fluid restrictions 420 are aligned in alternating rows and separated by about 6 millimeters. The spacing of the fluid restrictions 420 may vary in some embodiments to increase the density of the fluid restrictions 420 according to therapeutic requirements.

One or more of the components of the dressing 110 may additionally be treated with an antimicrobial agent in some embodiments. For example, the first layer 405 may be a foam, mesh, or non-woven coated with an antimicrobial agent. In some embodiments, the first layer 405 may comprise antimicrobial elements, such as fibers coated with an antimicrobial agent. Additionally or alternatively, some embodiments of the second layer 410 may be a polymer coated or mixed with an antimicrobial agent. In other examples, the fluid conductor 450 may additionally or alternatively be treated with one or more antimicrobial agents. Suitable antimicrobial agents may include, for example, metallic silver, PHMB, iodine or its complexes and mixes such as povidone iodine, copper metal compounds, chlorhexidine, or some combination of these materials.

Additionally or alternatively, one or more of the components may be coated with a mixture that may include citric acid and collagen, which can reduce bio-films and infections. For example, the first layer 405 may be foam coated with such a mixture.

Individual components of the dressing 110 may be bonded or otherwise secured to one another with a solvent or non-solvent adhesive, or with thermal welding, for example, without adversely affecting fluid management.

The cover 140, the first layer 405, and the second layer 410, or various combinations may be assembled before application or *in situ.* For example, the cover 140 may be laminated to the first layer 405, and the second layer 410 may be laminated to the first layer 405 opposite the cover 140 in some embodiments. The second layer 410 may provide a smooth surface opposite the first layer 405. In some embodiments, one or more layers of the tissue interface 135 may coextensive. For example, the second layer 410 may be cut flush with the edge of the first layer 405, exposing the edge of the first layer 405, as illustrated in the embodiment of Figure 4. In other embodiments, the second layer 410 may overlap the edge of the first layer 405. In some embodiments, the dressing 110 may be provided as a single, composite dressing. For example, the second layer 410 may be coupled to the cover 140 to enclose the first layer 405, wherein the second layer 410 is configured to face a tissue site.

In use, the release liner 445 (if included) may be removed to expose the second layer 410, which may be placed within, over, on, or otherwise proximate to a tissue site, particularly a surface tissue site and adjacent epidermis. The second layer 410 may be interposed between the first layer 405 and the tissue site and adjacent epidermis, which can substantially reduce or eliminate adverse interaction with the first layer 405. For example, the second layer 410 may be placed over a surface wound (including edges of the wound) and undamaged epidermis to prevent direct contact with the first layer 405. Treatment of a surface wound or placement of the dressing 110 on a surface wound includes placing the dressing 110 immediately adjacent to the surface of the body or extending over at least a portion of the surface of the body. Treatment of a surface wound does not include placing the dressing 110 wholly within the body or wholly under the surface of the body, such as placing a dressing within an abdominal cavity. The cover 140 may be sealed to an attachment surface, such as epidermis peripheral to a tissue site, around the first layer 405 and the second layer 410.

The geometry and dimensions of the tissue interface 135, the cover 140, or both may vary to suit a particular application or anatomy. For example, the geometry or dimensions of the tissue interface 135 and the cover 140 may be adapted to provide an effective and reliable seal against challenging anatomical surfaces, such as an elbow or heel, at and around a tissue site. Additionally or alternatively, the dimensions may be modified to increase the surface area for the second layer 410 to enhance the movement and proliferation of epithelial cells at a tissue site and reduce the likelihood of granulation tissue in-growth.

Thus, the dressing 110 in the example of Figure 4 can provide a sealed therapeutic environment proximate to a tissue site, substantially isolated from the external environment, and the negative-pressure source 105 can reduce the pressure in the sealed therapeutic environment.

The fluid mechanics of using a negative-pressure source to reduce pressure in another component or location, such as within a sealed therapeutic environment, can be mathematically complex. However, the basic principles of fluid mechanics applicable to negative-pressure therapy and instillation are generally well-known to those skilled in the art, and the process of reducing pressure may be described illustratively herein as "delivering," "distributing," or "generating" negative pressure, for example.

In general, exudate and other fluid flow toward lower pressure along a fluid path. Thus, the term "downstream" typically implies something in a fluid path relatively closer to a source of negative pressure or further away from a source of positive pressure. Conversely, the term "upstream" implies something relatively further away from a source of negative pressure or closer to a source of positive pressure. Similarly, it may be convenient to describe certain features in terms of fluid "inlet" or "outlet" in such a frame of reference. This orientation is generally presumed for purposes of describing various features and components herein. However, the fluid path may also be reversed in some applications (such as by substituting a positive-pressure source for a negative-pressure source) and this descriptive convention should not be construed as a limiting convention.

Negative pressure in the sealed environment may compress the first layer 405 into the second layer 410, which can deform the surface of the second layer 410 to provide an uneven, coarse, or jagged profile that can induce macrostrain and micro-strain in the tissue site in some embodiments. Negative pressure applied through the tissue interface 135 can also create a negative pressure differential across the fluid restrictions 420 in the second layer 410, which can open the fluid restrictions 420 to allow exudate and other liquid movement through the fluid restrictions 420 into the first layer 405 and the container 115. For example, in some embodiments in which the fluid restrictions 420 may comprise perforations through the second layer 410, a pressure gradient across the perforations can strain the adjacent material of the second layer 410 and increase the dimensions of the perforations to allow liquid movement through them, similar to the operation of a duckbill valve.

In some embodiments, the controller 120 may receive and process data from one or more sensors, such as the first sensor 125. The controller 120 may also control the operation of one or more components of the therapy system 100 to manage the pressure delivered to the tissue interface 135. In some embodiments, controller 120 may include an input for receiving a desired target pressure, and may be programmed for processing data relating to the setting and inputting of the target pressure to be applied to the tissue interface 135. In some example embodiments, the target pressure may be a fixed pressure value set by an operator as the target negative pressure desired for therapy at a tissue site and then provided as input to the controller 120. The target pressure may vary from tissue site to tissue site based on the type of tissue forming a tissue site, the type of injury or wound (if any), the medical condition of the patient, and the preference of the attending physician. After selecting a desired target pressure, the controller 120 can operate the negative-pressure source 105 in one or more control modes based on the target pressure, and may receive feedback from one or more sensors to maintain the target pressure at the tissue interface 135.

In some embodiments, the first layer 405 may be hydrophobic to minimize retention or storage of liquid in the dressing 110. In other embodiments, the first layer 405 may be hydrophilic to retain exudate or instillation solution. In an example in which the first layer 405 may be hydrophilic, the first layer 405 may also wick fluid away from a tissue site, while continuing to distribute negative pressure to the tissue site. The wicking properties of the first layer 405 may draw fluid away from a tissue site by capillary flow or other wicking mechanisms, for example. An example of a hydrophilic material that may be suitable for the first layer 405 is a polyvinyl alcohol, open-cell foam such as V.A.C. WHITEFOAM^{™} dressing available from KCI of San Antonio, Texas. Other hydrophilic foams may include those made from polyether. Other foams that may exhibit hydrophilic characteristics include hydrophobic foams that have been treated or coated to provide hydrophilicity.

If the negative-pressure source 105 is removed or turned-off, the pressure differential across the fluid restrictions 420 can dissipate, allowing the fluid restrictions 420 to return to an unstrained or resting state and prevent or reduce the return rate of exudate or other liquid moving to the tissue site through the second layer 410.

In some applications, a filler may also be disposed between a tissue site and the second layer 410. For example, if the tissue site is a surface wound, a wound filler may be applied interior to the periwound, and the second layer 410 may be disposed over the periwound and the wound filler. In some embodiments, the filler may be a manifold, such as open-cell foam. The filler may comprise or consist essentially of the same material as the first layer 405 in some embodiments.

Additionally or alternatively, instillation solution or other fluid may be distributed to the dressing 110, which can increase the pressure in the tissue interface 135. The increased pressure in the tissue interface 135 can create a positive pressure differential across the fluid restrictions 420 in the second layer 410, which can open or expand the fluid restrictions 420 from their resting state to allow the instillation solution or other fluid to be distributed to the tissue site.

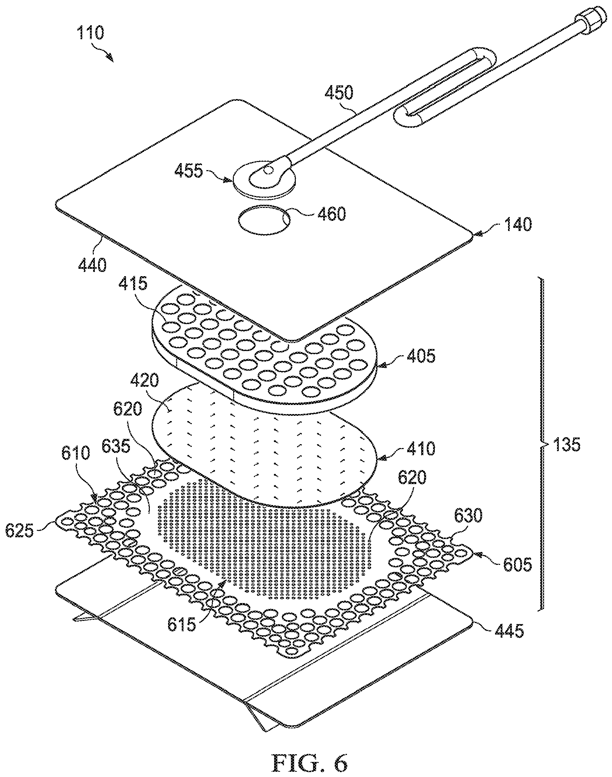

Figure 6 is an assembly view of another example of the dressing 110 of Figure 1, illustrating additional details that may be associated with some embodiments in which the tissue interface 135 may comprise additional layers. In the example of Figure 6, the tissue interface 135 comprises a third layer 605 in addition to the first layer 405 and the second layer 410. In some embodiments, the third layer 605 may be adjacent to the second layer 410 opposite the first layer 405. The third layer 605 may also be bonded to the second layer 410 in some embodiments.

The third layer 605 may comprise or consist essentially of a fixation layer having a tacky surface and formed from a soft polymer suitable for providing a fluid seal with a tissue site. The third layer may be a polymer gel having a coating weight of about 250 g.s.m., and may have a substantially flat surface in some examples. For example, the third layer 605 may comprise a silicone gel, a soft silicone, hydrocolloid, hydrogel, polyurethane gel, polyolefin gel, hydrogenated styrenic copolymer gel, a foamed gel, closed-cell foam such as polyurethanes and polyolefins coated with an adhesive, or acrylics. In some embodiments, the third layer 605 may have a thickness between about 200 microns (µm) and about 1000 microns (µm). In some embodiments, the third layer 605 may have a hardness between about 5 Shore OO and about 80 Shore OO. Further, the third layer 605 may be comprised of hydrophobic or hydrophilic materials.

In some embodiments, the third layer 605 may be a hydrophobic-coated material. For example, the third layer 605 may be formed by coating a spaced material, such as, for example, woven, nonwoven, molded, or extruded mesh with a hydrophobic material. The hydrophobic material for the coating may be a soft silicone, for example.

The third layer 605 may have a periphery 610 surrounding or around an interior portion 615, and may have apertures 620 disposed through the periphery 610 and the interior portion 615. The interior portion 615 may correspond to a surface area of the first layer 405 in some examples. The third layer 605 may also have corners 625 and edges 630. The corners 625 and the edges 630 may be part of the periphery 610. The third layer 605 may have an interior border 635 around the interior portion 615, disposed between the interior portion 615 and the periphery 610. The interior border 635 may be substantially free of the apertures 620, as illustrated in the example of Figure 6. In some examples, as illustrated in Figure 6, the interior portion 615 may be symmetrical and centrally disposed in the third layer 605.

The apertures 620 may be formed by cutting or by application of local RF or ultrasonic energy, for example, or by other suitable techniques for forming an opening. The apertures 620 may have a uniform distribution pattern, or may be randomly distributed on the third layer 605. The apertures 620 in the third layer 605 may have many shapes, including circles, squares, stars, ovals, polygons, slits, complex curves, rectilinear shapes, triangles, for example, or may have some combination of such shapes.

Each of the apertures 620 may have uniform or similar geometric properties. For example, in some embodiments, each of the apertures 620 may be circular apertures, having substantially the same diameter. In some embodiments, the diameter of each of the apertures 620 may be between about 1 millimeter to about 50 millimeters. In other embodiments, the diameter of each of the apertures 620 may be between about 1 millimeter to about 20 millimeters. In some embodiments, the diameter of each of the apertures 620 may be about 2 millimeters to about 5 millimeters.

In other embodiments, geometric properties of the apertures 620 may vary. For example, the diameter of the apertures 620 may vary depending on the position of the apertures 620 in the third layer 605, as illustrated in Figure 6. In some embodiments, the diameter of the apertures 620 in the periphery 610 of the third layer 605 may be larger than the diameter of the apertures 620 in the interior portion 615 of the third layer 605. For example, in some embodiments, the apertures 620 disposed in the periphery 610 may have a diameter between about 9.8 millimeters to about 10.2 millimeters. In some embodiments, the apertures 620 disposed in the corners 625 may have a diameter between about 7.75 millimeters to about 8.75 millimeters. In some embodiments, the apertures 62.0 disposed in the interior portion 615 may have a diameter between about 2 millimeters and about 5 millimeters.

At least one of the apertures 620 in the periphery 610 of the third layer 605 may be positioned at the edges 630 of the periphery 610, and may have an interior cut open or exposed at the edges 630 that is in fluid communication in a lateral direction with the edges 630. The lateral direction may refer to a direction toward the edges 630 and in the same plane as the third layer 605. As shown in the example of Figure 6, the apertures 620 in the periphery 610 may be positioned proximate to or at the edges 630 and in fluid communication in a lateral direction with the edges 630. The apertures 620 positioned proximate to or at the edges 630 may be spaced substantially equidistant around the periphery 610 as shown in the example of Figure 6. Alternatively, the spacing of the apertures 620 proximate to or at the edges 630 may be irregular.

As illustrated in the example of Figure 6, in some embodiments, the release liner 445 may be attached to or positioned adjacent to the third layer 605 to protect the adhesive 440 prior to use. In some embodiments, the release liner 445 may have a surface texture that may be imprinted on an adjacent layer, such as the third layer 605. Further, a release agent may be disposed on a side of the release liner 445 that is configured to contact the third layer 605.

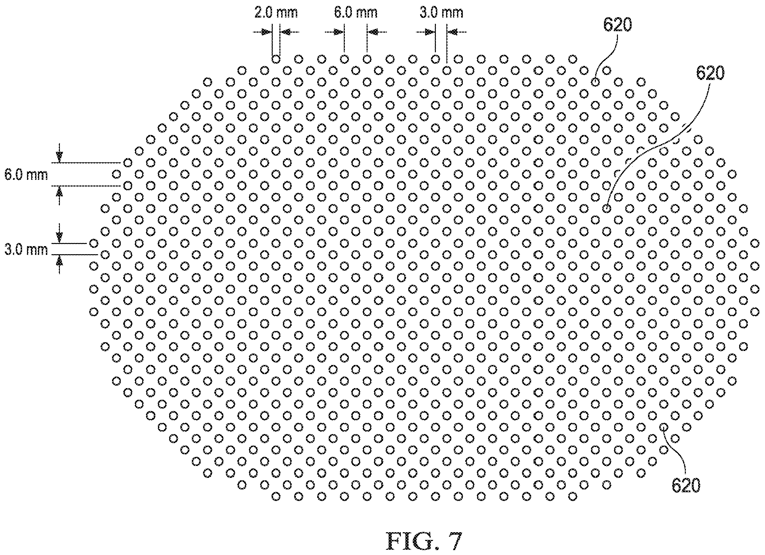

Figure 7 is a schematic view of an example configuration of the apertures 620, illustrating additional details that may be associated with some embodiments of the third layer 605. In some embodiments, the apertures 620 illustrated in Figure 7 may be associated only with the interior portion 615. In the example of Figure 7, the apertures 620 are generally circular and have a diameter of about 2 millimeters. Figure 7 also illustrates an example of a uniform distribution pattern of the apertures 620 in the interior portion 615. In Figure 7, the apertures 620 are distributed in a grid of parallel rows and columns. Within each row and column, the apertures 620 may be equidistant from each other, as illustrated in the example of Figure 7. Figure 7 illustrates one example configuration that may be particularly suitable for many applications, in which the apertures 620 are spaced about 6 millimeters apart along each row and column, with a 3 millimeter offset.

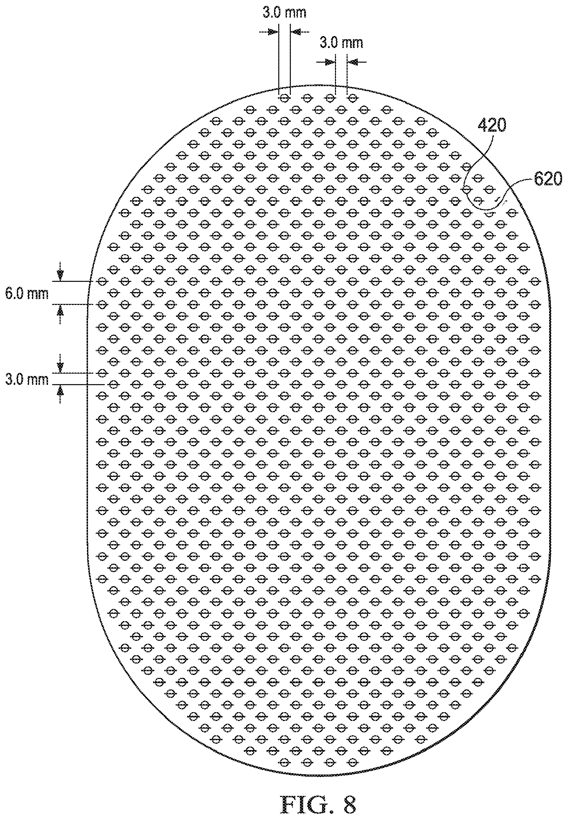

Figure 8 is a schematic view of the apertures 620 of Figure 7 overlaid on the fluid restrictions 420 of Figure 5, illustrating additional details that may be associated with some example embodiments of the tissue interface 135. For example, as illustrated in Figure 8, the fluid restrictions 420 may be aligned, overlapping, in registration with, or otherwise fluidly coupled to the apertures 620 in some embodiments. In some embodiments, one or more of the fluid restrictions 420 may be registered with the apertures 620 only in the interior portion 615, or only partially registered with the apertures 620. The fluid restrictions 420 in the example of Figure 8 are generally configured so that each of the fluid restrictions 420 is registered with only one of the apertures 620. In other examples, one or more of the fluid restrictions 420 may be registered with more than one of the apertures 620. For example, any one or more of the fluid restrictions 420 may be a perforation or a fenestration that extends across two or more of the apertures 620. Additionally or alternatively, one or more of the fluid restrictions 420 may not be registered with any of the apertures 620.

As illustrated in the example of Figure 8, the apertures 620 may be sized to expose a portion of the second layer 410, the fluid restrictions 420, or both through the third layer 605. In some embodiments, one or more of the apertures 435 may be sized to expose more than one of the fluid restrictions 420. For example, some or all of the apertures 435 may be sized to expose two or three of the fluid restrictions 420. In some examples, the length of each of the fluid restrictions 420 may be substantially equal to the diameter of each of the apertures 620. More generally, the average dimensions of the fluid restrictions 420 are substantially similar to the average dimensions of the apertures 620. For example, the apertures 620 may be elliptical in some embodiments, and the length of each of the fluid restrictions 420 may be substantially equal to the major axis or the minor axis. In some embodiments, though, the dimensions of the fluid restrictions 420 may exceed the dimensions of the apertures 620, and the size of the apertures 620 may limit the effective size of the fluid restrictions 420 exposed to the lower surface of the dressing 110.

Individual components of the dressing 110 in the example of Figure 6 may be bonded or otherwise secured to one another with a solvent or non-solvent adhesive, or with thermal welding, for example, without adversely affecting fluid management. Further, the second layer 410 or the first layer 405 may be coupled to the border 635 of the third layer 605 in any suitable manner, such as with a weld or an adhesive, for example.

The cover 140, the first layer 405, the second layer 410, the third layer 605, or various combinations may be assembled before application or *in situ.* For example, the cover 140 may be laminated to the first layer 405, and the second layer 410 may be laminated to the first layer 405 opposite the cover 140 in some embodiments. The third layer 605 may also be coupled to the second layer 410 opposite the first layer 405 in some embodiments. In some embodiments, one or more layers of the tissue interface 135 may coextensive. For example, the second layer 410, the third layer 605, or both may be cut flush with the edge of the first layer 405, exposing the edge of the first layer 405, as illustrated in the embodiment of Figure 6. In other embodiments, the second layer 410, the third layer 605, or both may overlap the edge of the first layer 405. In some embodiments, the dressing 110 may be provided as a single, composite dressing. For example, the third layer 605 may be coupled to the cover 140 to enclose the first layer 405 and the second layer 410, wherein the third layer 605 is configured to face a tissue site. Additionally or alternatively, the second layer 410, the third layer 605, or both may be disposed on both sides of the first layer 405 and bonded together to enclose the first layer 405.

Removing the release liner 445 in the example of Figure 6 can also expose the adhesive 440 and the cover 140 may be attached to an attachment surface, such as epidermis peripheral to a tissue site, around the first layer 405 and the second layer 410. For example, the adhesive 440 may be in fluid communication with an attachment surface through the apertures 620 in at least the periphery 610 of the third layer 605. The adhesive 440 may also be in fluid communication with the edges 630 through the apertures 620 exposed at the edges 630.

Once the dressing 110 is in the desired position, the adhesive 440 may be pressed through the apertures 620 to bond the dressing 110 to the attachment surface. The apertures 620 at the edges 630 may permit the adhesive 440 to flow around the edges 630 for enhancing the adhesion of the edges 630 to an attachment surface.

In some embodiments, apertures or holes in the third layer 605 may be sized to control the amount of the adhesive 440 in fluid communication with the apertures 620. For a given geometry of the corners 625, the relative sizes of the apertures 620 may be configured to maximize the surface area of the adhesive 440 exposed and in fluid communication through the apertures 620 at the corners 625. For example, as shown in Figure 6, the edges 630 may intersect at substantially a right angle, or about 90 degrees, to define the corners 625. In some embodiments, the corners 625 may have a radius of about 10 millimeters. Further, in some embodiments, three of the apertures 620 having a diameter between about 7.75 millimeters to about 8.75 millimeters may be positioned in a triangular configuration at the corners 625 to maximize the exposed surface area for the adhesive 440. In other embodiments, the size and number of the apertures 620 in the corners 625 may be adjusted as necessary, depending on the chosen geometry of the corners 625, to maximize the exposed surface area of the adhesive 440. Further, the apertures 620 at the corners 625 may be fully housed within the third layer 605, substantially precluding fluid communication in a lateral direction exterior to the corners 625. The apertures 620 at the corners 625 being fully housed within the third layer 605 may substantially preclude fluid communication of the adhesive 440 exterior to the corners 625, and may provide improved handling of the dressing 110 during deployment at a tissue site. Further, the exterior of the corners 625 being substantially free of the adhesive 440 may increase the flexibility of the corners 625 to enhance comfort.

In some embodiments, the bond strength of the adhesive 440 may vary in different locations of the dressing 110. For example, the adhesive 440 may have lower bond strength in locations adjacent to the third layer 605 where the apertures 620 are relatively larger, and may have higher bond strength where the apertures 620 are smaller. Adhesive 440 with lower bond strength in combination with larger apertures 620 may provide a bond comparable to adhesive 440 with higher bond strength in locations having smaller apertures 620.

The geometry and dimensions of the tissue interface 135, the cover 140, or both may vary to suit a particular application or anatomy. For example, the geometry or dimensions of the tissue interface 135 and the cover 140 may be adapted to provide an effective and reliable seal against challenging anatomical surfaces, such as an elbow or heel, at and around a tissue site. Additionally or alternatively, the dimensions may be modified to increase the surface area for the third layer 605 to enhance the movement and proliferation of epithelial cells at a tissue site and reduce the likelihood of granulation tissue in-growth.

Thus, the dressing 110 in the example of Figure 6 can provide a sealed therapeutic environment proximate to a tissue site, substantially isolated from the external environment, and the negative-pressure source 105 can reduce the pressure in the sealed therapeutic environment. The third layer 605 may provide an effective and reliable seal against challenging anatomical surfaces, such as an elbow or heel, at and around a tissue site. Further, the dressing 110 may permit re-application or re-positioning, to correct air leaks caused by creases and other discontinuities in the dressing 110, for example. The ability to rectify leaks may increase the efficacy of the therapy and reduce power consumption in some embodiments.

If not already configured, the dressing interface 455 may be disposed over the aperture 460 and attached to the cover 140. The fluid conductor 450 may be fluidly coupled to the dressing interface 455 and to the negative-pressure source 105.

Negative pressure applied through the tissue interface 135 can create a negative pressure differential across the fluid restrictions 420 in the second layer 410, which can open or expand the fluid restrictions 420. For example, in some embodiments in which the fluid restrictions 420 may comprise substantially closed fenestrations through the second layer 410, a pressure gradient across the fenestrations can strain the adjacent material of the second layer 410 and increase the dimensions of the fenestrations to allow liquid movement through them, similar to the operation of a duckbill valve. Opening the fluid restrictions 420 can allow exudate and other liquid movement through the fluid restrictions 420 into the first layer 405 and the container 115. Changes in pressure can also cause the first layer 405 to expand and contract, and the interior border 635 may protect the epidermis from irritation. The second layer 410 and the third layer 605 can also substantially reduce or prevent exposure of tissue to the first layer 405, which can inhibit growth of tissue into the first layer 405.

If the negative-pressure source 105 is removed or turned off, the pressure differential across the fluid restrictions 420 can dissipate, allowing the fluid restrictions 420 to close and prevent exudate or other liquid from returning to the tissue site through the second layer 410.

In some applications, a filler may also be disposed between a tissue site and the third layer 605. For example, if the tissue site is a surface wound, a wound filler may be applied interior to the periwound, and the third layer 605 may be disposed over the periwound and the wound filler. In some embodiments, the filler may be a manifold, such as open-cell foam. The filler may comprise or consist essentially of the same material as the first layer 405 in some embodiments.

Additionally or alternatively, instillation solution or other fluid may be distributed to the dressing 110, which can increase the pressure in the tissue interface 135. The increased pressure in the tissue interface 135 can create a positive pressure differential across the fluid restrictions 420 in the second layer 410, which can open the fluid restrictions 420 to allow the instillation solution or other fluid to be distributed to a tissue site.

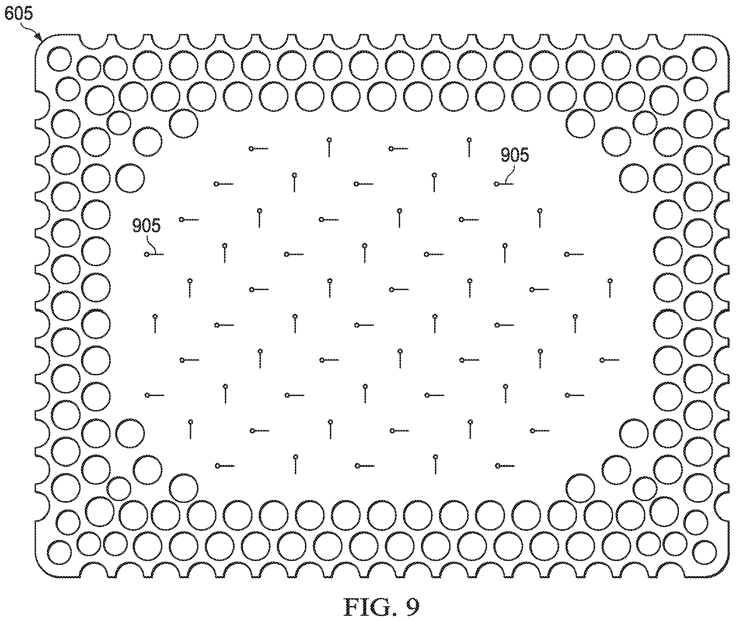

Figure 9 is a top view of another example the third layer 605, illustrating additional details that may be associated with some embodiments. As shown in the example of Figure 9, the third layer 605 may have one or more elastomeric valves 905 instead of or in addition to the apertures 620 in the interior portion 615. The valves 905 may be included in the third layer 605 in addition to or instead of the second layer 410. In some embodiments in which the third layer 605 includes one or more of the valves 905, the second layer 410 may be omitted. For example, in some embodiments, the tissue interface 135 may consist essentially of the first layer 405 and the third layer 605 of Figure 9 with the valves 905 disposed in the interior portion 615.

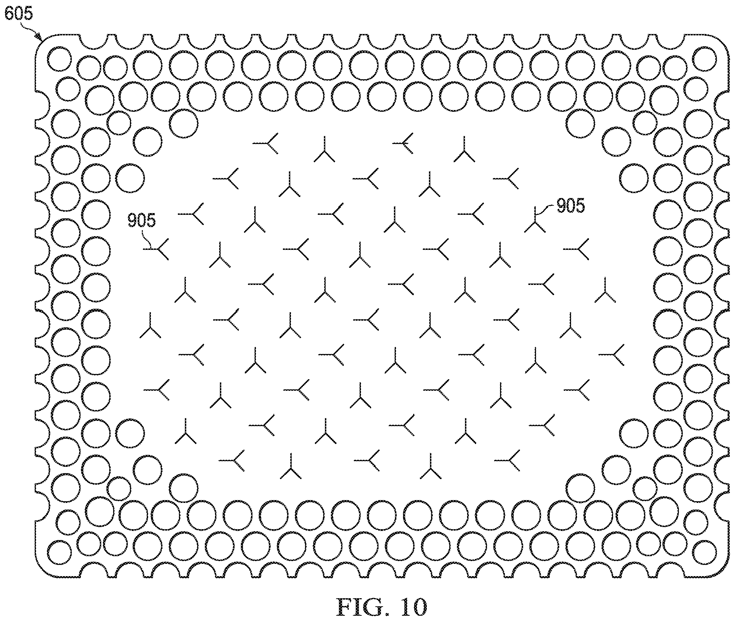

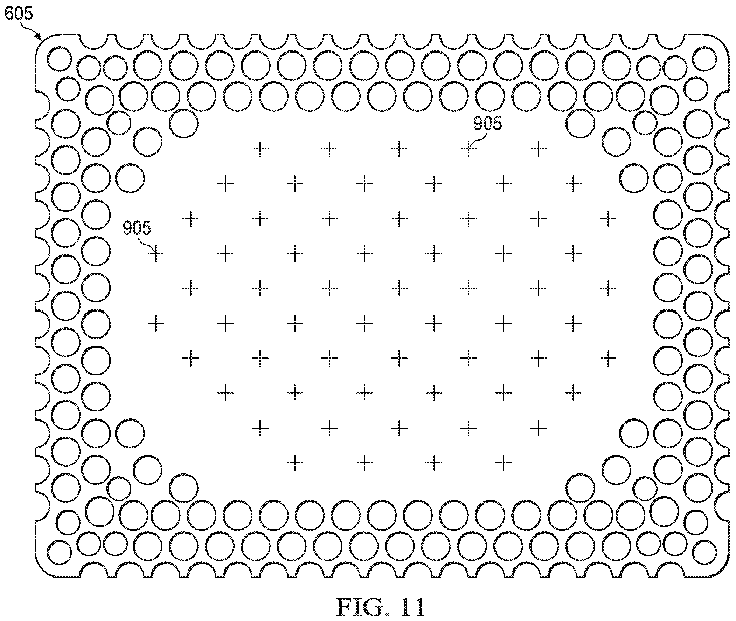

Figure 10 and Figure 11 illustrate other example configurations of the valves 905, in which the valves 905 each generally comprise a combination of intersecting slits or cross-slits.

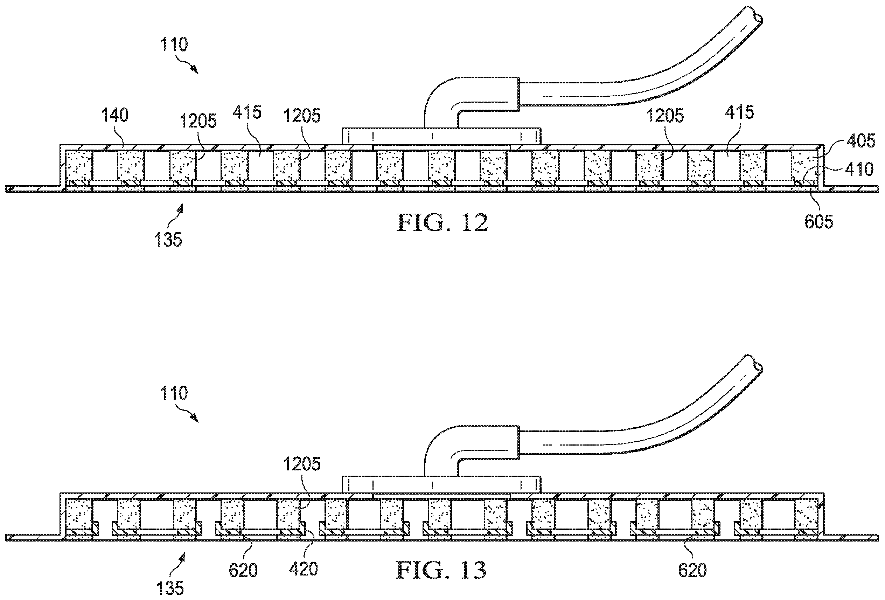

Figure 12 is a schematic section view of an example of the dressing 110, illustrating additional details that may be associated with some embodiments. In the example of Figure 12, the tissue interface 135 includes the first layer 405, the second layer 410, and the third layer 605 assembled in a stacked configuration in which the second layer 410 is disposed between the first layer 405 and the third layer 605. The cover 140 of Figure 12 is disposed over the first layer 405. The cover 140 substantially encloses the edges of the tissue interface 135.

As illustrated in the example configuration of Figure 12, the perforations 415 may extend through the first layer 405. One or more of the perforations 415 may be a through-hole that extends through the first layer 405 from a first surface adjacent to the cover 140 to a second surface adjacent to the second layer 410. In other embodiments, one or more of the perforations 415 may be a blind hole, which does not pass completely through the first layer 405. For example, one or more of the perforations 415 may extend into the first layer 405 from the first surface and may have a depth that is less than the thickness of the first layer 405.

The perforations 415 may form walls 1205 in the first layer 405. In some embodiments, the walls 1205 may be cylindrical. In still other embodiments, the perforations 415 may be tapered, and may have conical, pyramidal, or other irregular geometries.

Figure 13 is a schematic section view of another example configuration of the tissue interface 135. In the example of Figure 13, one or more of the fluid restrictions 420 may be configured to expand under a pressure gradient to contact at least a portion of the walls 1205. For example, one or more of the fluid restrictions 420 may comprise or consist of a cross-slit, which can form flaps that can contact at least a portion of the walls 1205. The flaps can prevent or reduce tissue growth into the first layer 405. Granulation tissue can also cause expansion of the fluid restrictions 420 in some examples. In some embodiments having the third layer 605, one or more of the fluid restrictions 420 may be aligned with one or more of the apertures 620.

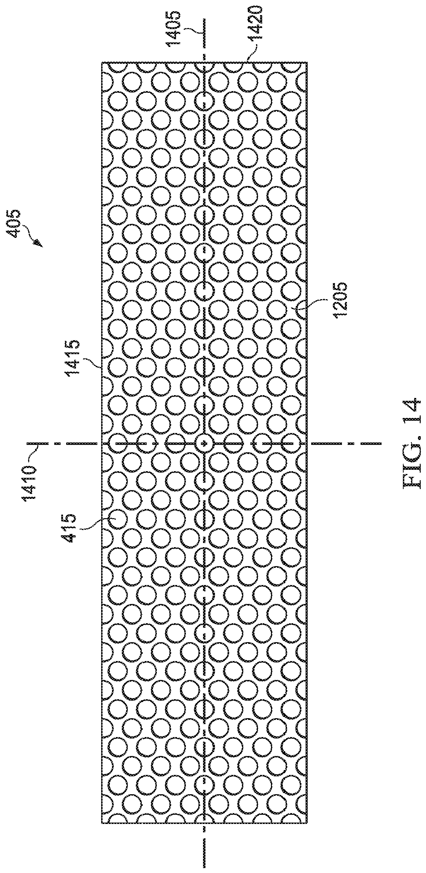

Figure 14 is a plan view of an example of the first layer 405, illustrating additional details that may be associated with some embodiments. For example, some embodiments of the perforations 415 may have a circular cross-section as illustrated in Figure 14. In some embodiments, the perforations 415 may have an average diameter of about 2 millimeters to about 10 millimeters, and centers of the perforations 415 may be spaced between about 2 millimeters and about 10 millimeters. The perforations 415 may be aligned with all, some, or none of the fluid restrictions 420 and the apertures 620.

In some embodiments, the first layer 405 may have a first orientation line 1405 and a second orientation line 1410 that is perpendicular to the first orientation line 1405. The first orientation line 1405 and the second orientation line 1410 may be lines of symmetry through the first layer 405. In the example of Figure 14, the first layer 405 has a generally rectangular shape with longitudinal edges 1415 and latitudinal edges 1420. In some embodiments, the first orientation line 1405 may be parallel to the longitudinal edges 1415.

In some embodiments, the longitudinal edges 1415 and the latitudinal edges 1420 of the first layer 405 may not be straight edges. For example, one or more of the perforations 415 may overlap the longitudinal edges 1415 or the latitudinal edges 1420, causing the edge to have a non-linear profile, which may reduce the disruption of keratinocyte migration and enhance re-epithelialization with negative-pressure therapy.

The first layer 405 may also have a variety of other suitable shapes. For example, the first layer 405 may have a diamond, square, or circular shape. In some embodiments, the shape of the first layer 405 may be selected to accommodate the shape or type of a tissue site. For example, the first layer 405 may have an oval or circular shape to accommodate an oval or circular tissue site.

In some embodiments, the perforations 415 may be aligned in parallel rows to form an array, as illustrated in the example of Figure 14. In some embodiments, a width of the walls 1205 between the perimeter of two or more of the perforations 415 in a row may be about 5 millimeters. The center of each of the perforations 415 in adjacent rows may be characterized as being offset along the first orientation line 1405.

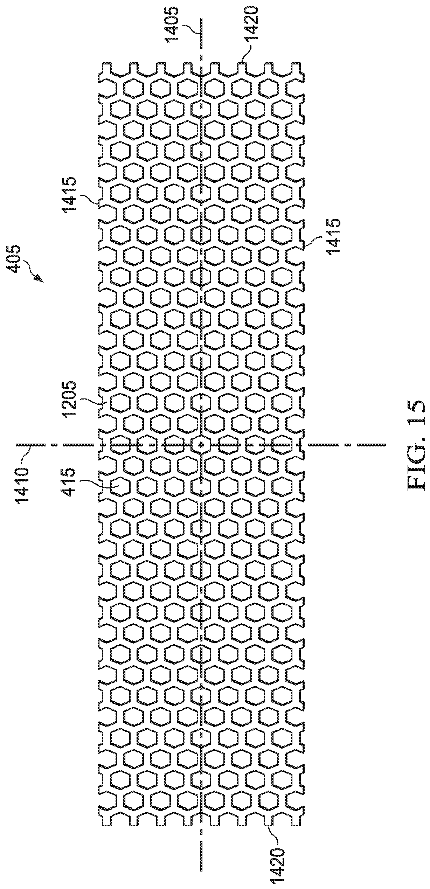

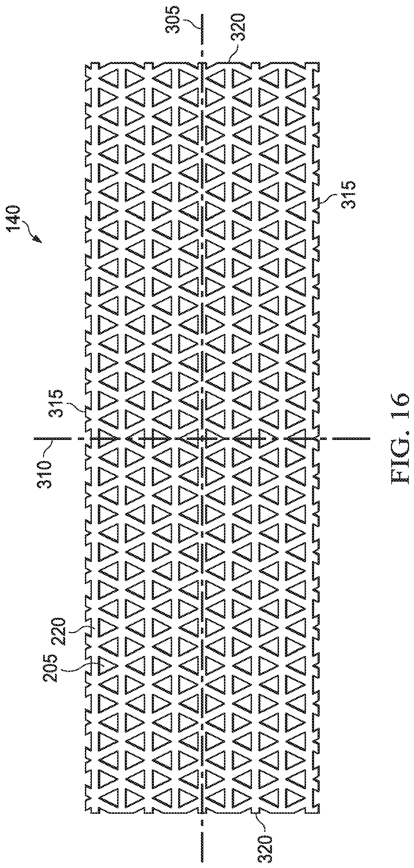

Figure 15 and Figure 16 are plan views of other examples of the first layer 405, illustrating additional details that may be associated with some embodiments. As illustrated in Figure 15 and Figure 16, some embodiments of the perforations 415 may have a polygonal cross-section. Figure 15 illustrates an example in which each of the perforations has a hexagonal cross-section. Figure 16 illustrates an example in which each of the perforations has a triangular cross-section. In some embodiments, the perforations 415 may have an effective diameter between about 2 millimeters and about 10 millimeters. An effective diameter of a non-circular area is a diameter of a circular area having the same surface area as the non-circular area.

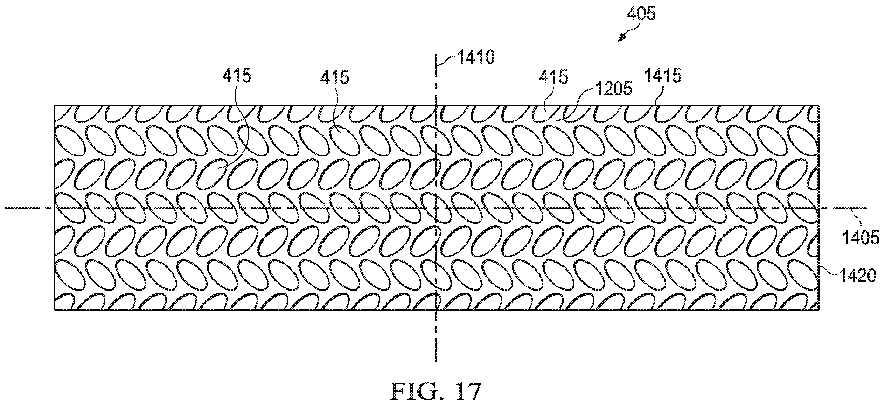

Figure 17 is a plan view of another example of the first layer 405, illustrating additional details that may be associated with some embodiments. In the example of Figure 17, each of the perforations 415 has an elliptical cross-section.

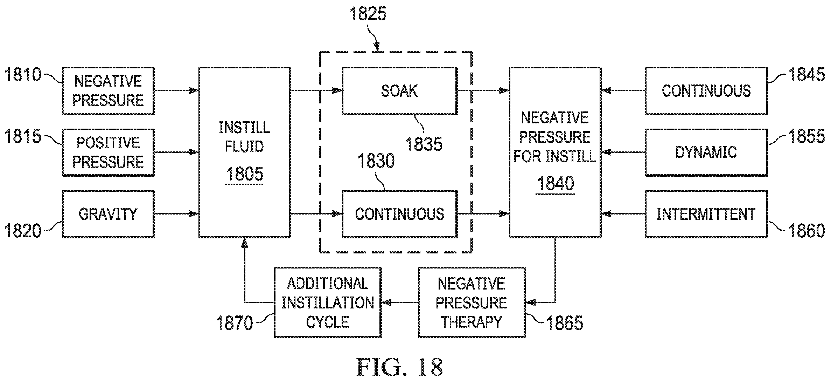

Figure 18 is a chart illustrating details that may be associated with an example method of operating the therapy system 100 to provide negative-pressure treatment and instillation treatment to the tissue interface 135. In some embodiments, the controller 120 may receive and process data, such as data related to instillation solution provided to the tissue interface 135. Such data may include the type of instillation solution prescribed by a clinician, the volume of fluid or solution to be instilled to a tissue site ("fill volume"), and the amount of time prescribed for leaving solution at a tissue site ("dwell time") before applying a negative pressure to the tissue site. The fill volume may be, for example, between 10 and 500 mL, and the dwell time may be between one second to 30 minutes. The controller 120 may also control the operation of one or more components of the therapy system 100 to instill solution, as indicated at 1805. For example, the controller 120 may manage fluid distributed from the solution source 145 to the tissue interface 135. In some embodiments, fluid may be instilled to a tissue site by applying a negative pressure from the negative-pressure source 105 to reduce the pressure at the tissue site, drawing solution into the tissue interface 135, as indicated at 1810. In some embodiments, solution may be instilled to a tissue site by applying a positive pressure from the positive-pressure source 150 to move solution from the solution source 145 to the tissue interface 135, as indicated at 1815. Additionally or alternatively, the solution source 145 may be elevated to a height sufficient to allow gravity to move solution into the tissue interface 135, as indicated at 1820.

The controller 120 may also control the fluid dynamics of instillation at 1825 by providing a continuous flow of solution at 1830 or an intermittent flow of solution at 1835. Negative pressure may be applied to provide either continuous flow or intermittent flow of solution at 1840. The application of negative pressure may be implemented to provide a continuous pressure mode of operation at 1845 to achieve a continuous flow rate of instillation solution through the tissue interface 135, or may provide a dynamic pressure mode of operation at 1855 to vary the flow rate of instillation solution through the tissue interface 135. Alternatively, the application of negative pressure may be implemented to provide an intermittent mode of operation at 1860 to allow instillation solution to dwell at the tissue interface 135. In an intermittent mode, a specific fill volume and dwell time may be provided depending, for example, on the type of tissue site being treated and the type of dressing being utilized. After or during instillation of solution, negative-pressure treatment may be applied at 1865. The controller 120 may be utilized to select a mode of operation and the duration of the negative pressure treatment before commencing another instillation cycle at 1870 by instilling more solution at 1805.

The systems, apparatuses, and methods described herein may provide significant advantages. The dressing 110 may be applied over intact skin with no sizing, and can reduce risk of maceration. In some configurations, the dressing 110 may be left on a wound for up to seven days with low risk of tissue in-growth or loss of manifold effectiveness. The dressing 110 may be particularly advantageous for use with treatment of moderate depth wounds with medium-to-high levels of exudate. For example, some wounds, particularly those that are highly infected, may produce very viscous exudate which can reduced the level of negative pressure delivered to a wound site. The perforations 415 can increase efficacy of exudate removal through the dressing 110. Moreover, wounds producing thick exudate may also be treated with instillation therapy to treat causal infection, to disburse exudate from the wound and dressing, or both. The perforations 415 can increase the efficiency of applied instillation solution to both remove exudate from the dressing 110 and deliver solution to the wound.

While shown in a few illustrative embodiments, a person having ordinary skill in the art will recognize that the systems, apparatuses, and methods described herein are susceptible to various changes and modifications that fall within the scope of the appended claims. Moreover, descriptions of various alternatives using terms such as "or" do not require mutual exclusivity unless clearly required by the context, and the indefinite articles "a" or "an" do not limit the subject to a single instance unless clearly required by the context. Components may be also be combined or eliminated in various configurations for purposes of sale, manufacture, assembly, or use. For example, in some configurations the dressing 110, the container 115, or both may be eliminated or separated from other components for manufacture or sale. In other example configurations, the controller 120 may also be manufactured, configured, assembled, or sold independently of other components.

The appended claims set forth novel and inventive aspects of the subject matter described above, but the claims may also encompass additional subject matter not specifically recited in detail. For example, certain features, elements, or aspects may be omitted from the claims if not necessary to distinguish the novel and inventive features from what is already known to a person having ordinary skill in the art. Features, elements, and aspects described in the context of some embodiments may also be omitted, combined, or replaced by alternative features serving the same, equivalent, or similar purpose without departing from the scope of the invention defined by the appended claims.

Aspects of the invention are disclosed in the following numbered clauses:
1. A dressing for treating a tissue site with negative pressure, the dressing comprising:
   a first layer comprising a manifold having a plurality of through-holes;
   a second layer adjacent to the first layer, the second layer comprising a polymer film having a plurality of fluid restrictions that are configured to expand in response to a pressure gradient across the polymer film; and
   a cover adjacent to the first layer, the cover comprising a polymer drape.
2. The dressing of clause 1, wherein each of the plurality of through-holes has an effective diameter of at least 2 millimeters.
3. The dressing of clause 1, wherein each of the plurality of through-holes has an effective diameter not greater than 10 millimeters.
4. The dressing of clause 1, wherein each of the plurality of through-holes has an effective diameter between about 2 millimeters and about 10 millimeters.
5. The dressing of any one of clauses 1-4, wherein the plurality of through-holes are spaced about 2 millimeters on center.
6. The dressing of any one of clauses 1-4, wherein the plurality of through-holes are spaced about 10 millimeters on center.
7. The dressing of any one of clauses 1-4, wherein the plurality of through-holes are spaced between about 2 millimeters and about 10 millimeters on center.
8. The dressing of any preceding clause, wherein the plurality of through-holes are formed in an array.
9. The dressing of any preceding clause, wherein the through-holes are offset from the fluid restrictions.
10. The dressing of any preceding clause, wherein at least some of the through-holes are registered with at least some of the fluid restrictions.
11. The dressing of any preceding clause, wherein the manifold comprises reticulated foam.
12. The dressing of clause 12, wherein the manifold comprises reticulated foam that is compressed.
13. The dressing of clause 12, wherein the manifold comprises reticulated foam that is felted.
14. The dressing of any preceding clause, wherein the polymer film of the second layer is hydrophobic.
15. The dressing of clause 14, wherein the polymer film of the second layer is polyethylene.
16. The dressing of clause 14, wherein the polymer film is polyethylene film having an area density of less than 30 grams per square meter.
17. The dressing of any one of clauses 1-16, wherein the fluid restrictions comprise a plurality of slots, each of the slots having a length less than 5 millimeters.
18. The dressing of any one of clauses 1-16, wherein the fluid restrictions comprise a plurality of slots, each of the slots having a width less than 2 millimeters.
19. The dressing of any of one of clauses 1-16, wherein the fluid restrictions comprise a plurality of slots, each of the slots having a length less than 4 millimeters and a width less than 2 millimeters.
20. The dressing of any preceding clause, wherein the fluid restrictions comprise or consist essentially of elastomeric valves in the polymer film, and the elastomeric valves are normally closed.
21. The dressing of any one of clauses 1-16, wherein the fluid restrictions comprise fenestrations in the polymer film.
22. The dressing of clause 21, wherein the fluid restrictions comprise slits in the polymer film.
23. The dressing of clause 21, wherein the fluid restrictions comprise intersecting slits in the polymer film.
24. The dressing of clause 21, wherein the fluid restrictions comprise flaps configured to at least partially open into the through-holes under negative pressure.
25. The dressing of any preceding clause, wherein the cover comprises a margin that extends beyond the first layer and the second layer, and an adhesive disposed in the margin.
26. The dressing of any preceding clause, further comprising a third layer disposed adjacent to the second layer, the third layer comprising a gel having a plurality of apertures.
27. The dressing of clause 26, wherein the gel of the third layer is a polymer gel having a coat weight of about 250 grams per square centimeter.
28. The dressing of clause 26, wherein the gel of the third layer is a silicone gel.
29. The dressing of clause 26, wherein the gel of the third layer comprises a silicone gel having a coat weight of about 2.50 grams per square centimeter.
30. The dressing of clause 26, wherein the gel is silicone, polyurethane, hydrocolloid, or acrylic.
31. The dressing of clause 26, wherein the plurality of apertures of the third layer have an effective diameter of about 2 millimeters to about 5 millimeters.
32. The dressing of clause 26, wherein the first layer and the second layer are enclosed between the third layer and the cover.
33. The dressing of clause 26, wherein at least some of the plurality of apertures are registered with at least some of the fluid restrictions.
34. The dressing of clause 26, wherein the through-holes are registered with the fluid restrictions and the plurality of apertures.
35. The dressing of clause 26, wherein the fluid restrictions have an average length that does not substantially exceed an average dimension of the apertures.
36. The dressing of clause 26, wherein the apertures limit an effective size of the fluid restrictions.
37. The dressing of clause 26, wherein each of the apertures are sized to expose no more than two of the fluid restrictions.
38. The dressing of any preceding clause, wherein the polymer film does not extend into the through-holes.
39. The dressing of any preceding clause, wherein the polymer film does not extend across the through-holes.
40. The dressing of any preceding clause, further comprising a fluid port coupled to the cover, the fluid port configured to be coupled to a fluid conductor.
41. A system for treating tissue with negative pressure, the system comprising:
   a negative-pressure source; and
   a dressing according to any of clauses 1-40 fluidly coupled to the negative-pressure source.
42. The system of clause, 41, further comprising a source of instillation solution fluidly coupled to the dressing.
43. A method of treating a wound with negative pressure, the method comprising:
   applying a dressing according to any of clauses 1-40 to the wound;
   fluidly coupling a negative-pressure source to the dressing; and
   delivering a therapeutic level of negative pressure from the negative-pressure source to the dressing.
44. The method of clause 43, further comprising:
   fluidly coupling a source of instillation solution to the dressing; and
   delivering solution from the source of instillation solution to the dressing.
45. The method of clause 43 or clause 44, wherein applying the dressing comprises sealing the dressing to epidermis adjacent to the wound.
46. The method of any one of clauses 43-45, wherein applying the dressing comprises disposing at least part of the dressing across an edge of the wound.
47. The method of any one of clauses 43-46, wherein the wound is a surface wound.
48. The method of any one of clauses 43-47, wherein the dressing remains on the wound for at least 7 days.
49. Use of any of the dressings, systems, or methods of any preceding clause to treat a tissue site.
50. Use of any of the dressings, systems, or methods of any preceding clause to treat a wound.
51. Use of any of the dressings, systems, or methods of any preceding clause to treat a wound for at least 7 days without changing the dressing.
52. The systems, apparatuses, and methods substantially as described herein.

## Claims

1. A dressing for treating a tissue site with negative pressure, the dressing comprising:
a first layer comprising an open-cell foam manifold;
a second layer adjacent to the first layer, the second layer comprising a polymer film having a plurality of fluid restrictions that are configured to expand in response to a pressure gradient across the polymer film;
a third layer disposed adjacent to the second layer, the third layer comprising a gel having a plurality of apertures; and
a cover adjacent to the first layer, the cover comprising a polymer drape.

2. The dressing of claim 1, wherein the manifold comprises reticulated foam.

3. The dressing of any preceding claim, wherein the polymer film of the second layer is hydrophobic, optionally wherein the polymer film of the second layer is polyethylene, optionally wherein the polymer film is polyethylene film having an area density of less than 30 grams per square meter.

4. The dressing of any preceding claim, wherein the fluid restrictions comprise a plurality of slots, each of the slots having a length less than 4 millimeters and a width less than 2 millimeters.

5. The dressing of any preceding claim, wherein the fluid restrictions comprise or consist essentially of elastomeric valves in the polymer film, and the elastomeric valves are normally closed.

6. The dressing of any preceding claim, wherein the fluid restrictions comprise fenestrations in the polymer film, optionally wherein the fluid restrictions comprise slits in the polymer film, optionally wherein the fluid restrictions comprise intersecting slits in the polymer film.

7. The dressing of any preceding claim, wherein the cover comprises a margin that extends beyond the first layer and the second layer, and an adhesive disposed in the margin.

8. The dressing of any preceding claim, wherein the plurality of apertures of the third layer are disposed through a periphery and an interior portion of the third layer, and wherein one or more of the plurality of apertures in the interior portion of the third layer is sized such that a portion of the second layer extends freely across at least one of the apertures in the third layer.

9. The dressing of any preceding claim, wherein one or more of the fluid restrictions of the second layer includes a length that extends across an edge of one or more of the apertures of the third layer.

10. The dressing of any preceding claim, wherein the gel is silicone, polyurethane, hydrocolloid, or acrylic.

11. The dressing of any preceding claim, wherein the first layer and the second layer are enclosed between the third layer and the cover.

12. The dressing of any preceding claim, wherein at least some of the plurality of apertures are registered with at least some of the fluid restrictions.

13. The dressing of any preceding claim, wherein at least one of the apertures are sized to expose more than one of the fluid restrictions.

14. A system for treating tissue with negative pressure, the system comprising:
a negative-pressure source; and
a dressing according to any of claims 1-13 fluidly coupled to the negative-pressure source.

15. The system of claim 14, further comprising a source of instillation solution fluidly coupled to the dressing.
